# FASCICULE DE BREVET EUROPEEN

(11) **EP 1 307 161 B1**
(45) Date de publication et mention de la délivrance du brevet: **29.08.2007**
(21) Numéro de dépôt: 01929725.8
(22) Date de dépôt: 26.04.2001
(51) Int. Cl.: A61F 2/00

(54) **PAROI DE RENFORT POUR LE TRAITEMENT DE TISSUS ALTERES DE LA PAROI ABDOMINALE**
VERSTEIFUNGSWAND ZUR BEHANDLUNG VON GESCHÄDIGTEM GEWEBE DER BAUCHWAND
REINFORCING WALL FOR TREATING DAMAGED ABDOMINAL WALL TISSUES

(30) Priorité: 26.04.2000 FR 0005562
(43) Date de publication de la demande: 07.05.2003
(73) Titulaire: Aspide, 42350 La Talaudière (FR)
(72) Inventeur: BOUILLET, Valérie-France, F-42350 La Talaudière (FR)
(74) Mandataire: Martin, Didier Roland Valéry
(86) Numéro de dépôt international: PCT/FR2001/001292
(87) Numéro de publication internationale: WO 2001/080774

(56) Documents cités:
- WO-A-98/07384
- WO-A-99/16381
- FR-A- 2 737 106
- RICCARDO PIETRABISSA: "Biomateriali per protesi e organi artificiali" 1996, PÀTRON EDITORE , BOLOGNA * page 157 - page 158 *

## Description

### DOMAINE TECHNIQUE

La présente invention se rapporte au domaine technique général des plaques de renfort pour le traitement de tissus altérés de la paroi abdominale d'un corps humain.

Plus particulièrement, la présente invention est relative à une plaque de renfort pour le traitement de tissus altérés de la paroi abdominale, formée d'un matériau non tissé à base de polypropylène et implantée dans le corps d'un patient par chirurgie.

La présente invention est notamment relative au traitement des hernies. Une hernie consiste en une saillie permanente ou intermittente de viscères, habituellement intestinaux, au travers d'un point de faiblesse de la paroi abdominale. La présente invention est également relative au traitement des éventrations, ou bien encore des prolapsus rectaux et génitaux urinaires.

### TECHNIQUE ANTERIEURE

Pour éviter l'étranglement des viscères au travers d'une hernie, il est nécessaire de réduire chirurgicalement la hernie par un traitement qui soit aussi définitif que possible. Lorsque la taille et la qualité des tissus le permettent, des fils de suture suffisent. Toutefois, lorsque l'orifice de la hernie est trop grand ou lorsque les tissus sont trop fragiles, il est nécessaire de rapporter une plaque de renfort pour combler la perte de substance.

Les plaques de renfort sont implantées selon deux techniques chirurgicales distinctes, à savoir par laparotomie et par laparoscopie.

Lorsque la technique de laparotomie est utilisée, le chirurgien réalise une large ouverture au niveau de l'abdomen afin d'implanter manuellement la plaque de renfort après avoir replacé l'intestin dans sa position normale.

Par la technique de laparoscopie, le chirurgien réalise de petites ouvertures de l'abdomen en pratiquant de petites incisions à des niveaux différents, afin de lui permettre d'insérer les différents instruments et une petite caméra pour visualiser l'intérieur de l'abdomen et localiser la hernie. L'abdomen est gonflé avec un gaz et la plaque de renfort est introduite au travers d'un tube (trocart) pour être ensuite immobilisée par agrafage ou sutures, ou bien encore clips.

Parmi les plaques de renfort habituellement rencontrées, certaines sont réalisées par tissage ou tricotage d'un ou plusieurs filaments de polypropylène. Même si ces plaques permettent une bonne colonisation des tissus au travers des mailles de la plaque, ainsi qu'une bonne visibilité de la zone d'implantation pour le chirurgien lors de l'opération, elles créent des gènes et peuvent être désagréablement perçues par le patient.

Pour remédier à ces inconvénients, on a déjà proposé des plaques réalisées en un matériau polypropylène non tissé. Ces plaques de renfort sont pleines, de sorte que le chirurgien ne peut pas apercevoir la zone d'implantation et la colonisation des tissus est dégradée.

On connaît également du document. WO-99/16381 une plaque de renfort correspondant au préambule de la revendication 1.

### EXPOSE DE L'INVENTION

Les objets assignés à la présente invention visent en conséquence à proposer une nouvelle plaque de renfort ne présentant pas les différents inconvénients énumérés précédemment et qui permette un traitement simple de la paroi abdominale tout en évitant la récidive des hernies.

Un autre objet de l'invention vise à proposer une nouvelle plaque de renfort capable d'assurer un bon ancrage de la plaque par les tissus.

Encore un autre objet de la présente invention vise à proposer une nouvelle plaque de renfort dont la pose soit facilitée.

Un autre objet de la présente invention vise à proposer une nouvelle plaque de renfort qui minimise les risques d'erreurs de l'agrafage et/ou de la suture de la plaque in situ.

Les objets assignés à la présente invention sont atteints à l'aide d'une plaque de renfort conforme au libellé de la revendication 1.

### DESCRIPTIF SOMMAIRE DES DESSINS

Les objets assignés à l'invention seront explicités plus en détails à la lecture de la description qui suit à l'aide des dessins annexés ci-après, donnés à titre purement illustratif et non limitatif, dans lesquels :
- La figure 1 illustre, selon une vue en coupe transversale partielle, une hernie pouvant être traitée à l'aide d'une plaque de renfort selon la présente invention.
- La figure 2 illustre, selon une vue de dessus, une plaque de renfort selon un premier mode de réalisation de la présente invention.
- Les figures 3 et 4 caractérisent l'implantation des différentes perforations portées par la plaque de renfort de la figure 1.
- La figure 5 illustre, selon une vue de dessus, une plaque de renfort selon un deuxième mode de réalisation de la présente invention.

### MEILLEURE MANIERE DE REALISER L'INVENTION

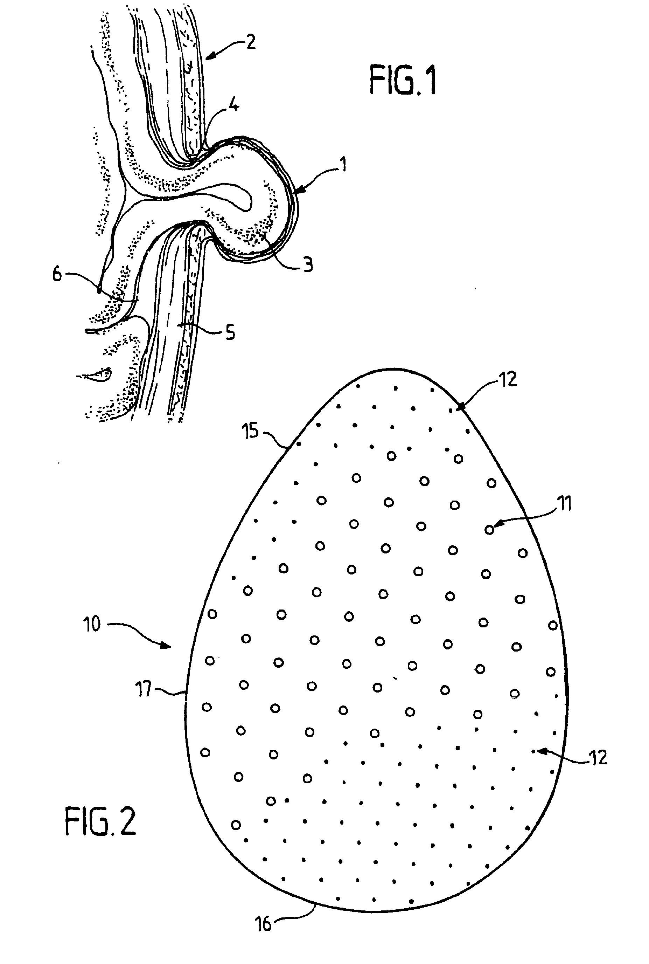

La figure 1 représente en coupe transversale une hernie 1 qui est formée par exemple dans la paroi abdominale 2 et qui est destinée à être résorbée au moyen de la paroi de renfort selon la présente invention.

La hernie 1 forme une grosseur constituée par exemple par une partie de l'intestin grêle 3 qui fait saillie au travers de l'orifice 4 apparu dans des tissus altérés 5 tels que des muscles, de la paroi abdominale 2. La hernie 1 représentée est par exemple localisée au niveau de l'aine du patient et est alors appelée hernie inguinale.

La plaque de renfort 10 selon la présente invention représentée à la figure 2 permet de réduire la hernie 1.

A cet effet, la plaque de renfort 10 est implantée au niveau de l'orifice 4 correspondant aux tissus altérés 5 de la paroi abdominale 2 par toutes techniques chirurgicales connues telles que celles précédemment évoquées, à savoir la laparotomie et la laparoscopie.

La plaque de renfort 10 est constituée d'une feuille réalisée en un matériau non tissé à base de polypropylène. Cette feuille forme par construction une surface pleine et est constituée d'une seule couche de fibres de polypropylène qui sont soudées entre elles et compressées.

Selon une caractéristique essentielle de la présente invention, des perforations 11 sont réalisées dans l'épaisseur de la plaque de renfort 10 pour favoriser la colonisation cellulaire et la reconstitution des tissus altérés.

Selon une caractéristique importante de l'invention, dans la plaque de renfort 10 ont été en outre ménagées des perforations 12 qui favorisent la mise en place de la plaque en regard des tissus altérés 5 de la paroi abdominale 2.

Les perforations de colonisation 11 sont de préférence de dimensions différentes des dimensions des perforations de mise en place 12, et encore de manière préférentielle sont de dimensions supérieures.

Les perforations des colonisations 11 sont destinées à favoriser l'ancrage de la plaque 10 lorsque celle-ci a été positionnée en regard de l'orifice 4 des tissus altérés 5, après que la hernie 1 ait été repoussée dans la cavité abdominale 6 par le chirurgien. Les tissus altérés 5 se développent ainsi de part et d'autre de la plaque de renfort 10, de sorte qu'après une durée de quinze jours, cette plaque s'est totalement intégrée à la paroi abdominale 2. La faiblesse des tissus est ainsi compensée par la présence de la plaque de renfort 10.

Les perforations de mise en place 12 sont adaptées pour que le chirurgien distingue, au travers de ces orifices, l'emplacement de la zone dans laquelle la plaque 10 doit être positionnée. Ces perforations matérialisent en outre la zone dans laquelle les agrafes ou les points de suture doivent être réalisés par le chirurgien au cours de l'opération. Ceci évite ainsi toute erreur de positionnement et facilite le travail du chirurgien en minimisant les risques opératoires.

Afin de favoriser l'aspect atraumatique de la plaque de renfort 10, cette plaque possède des bords arrondis et est de préférence de forme ovale. Elle comporte ainsi une portion supérieure rétrécie 15, une portion inférieure élargie 16 ainsi qu'une portion centrale 17 qui est située entre les parties supérieure 15 et inférieure 16. La surface de la portion supérieure 15 est plus petite que la surface de la portion inférieure 16. De préférence, la surface de la portion intermédiaire 17 est sensiblement égale à la surface des portions supérieure 15 et inférieure 16 réunies.

Les perforations de mise en place 12 sont situées dans la portion supérieure 15 et dans la portion inférieure 16. Les perforations de colonisation 11 sont situées dans la portion intermédiaire 17.

De manière préférentielle, les perforations de colonisation 11 et les perforations de mise en place 12 sont sensiblement chacune uniformément réparties dans chacune des portions.

De préférence, la densité des perforations de mise en place 12 est supérieure à la densité des perforations de colonisation 11.

Les lignes de démarcation fictives entre chacune des trois portions ont sensiblement la forme de droites qui s'étendent obliquement par rapport à l'axe de symétrie longitudinal de la plaque de renfort 10.

Comme cela est représenté à la figure 2, la portion supérieure 15 dans laquelle s'étendent des perforations de mise en place 12 possède une forme en fer à cheval dont la courbure est peu marquée.

Selon une autre caractéristique de l'invention, les perforations de colonisation 11 sont de forme sensiblement ovale ou oblongue, tandis que les perforations de mise en place 12 sont de forme sensiblement circulaire.

Dans un exemple préférentiel de l'invention, la plaque de renfort 10 possède une largeur (plus petite dimension) voisine de 100 mm et une hauteur (plus grande dimension) voisine de 140 mm. En variante, la largeur de la plaque peut être de l'ordre de 90 ou 110 mm et la hauteur de l'ordre de 130 ou 150 mm.

Dans tous les cas, les perforations de colonisation 11 sont constituées par des trous oblongs dont la largeur est de 1,5 mm et la hauteur de 2 mm, tandis que les perforations de mise en place 12 sont constituées par des trous de diamètre égal à 0,5 mm.

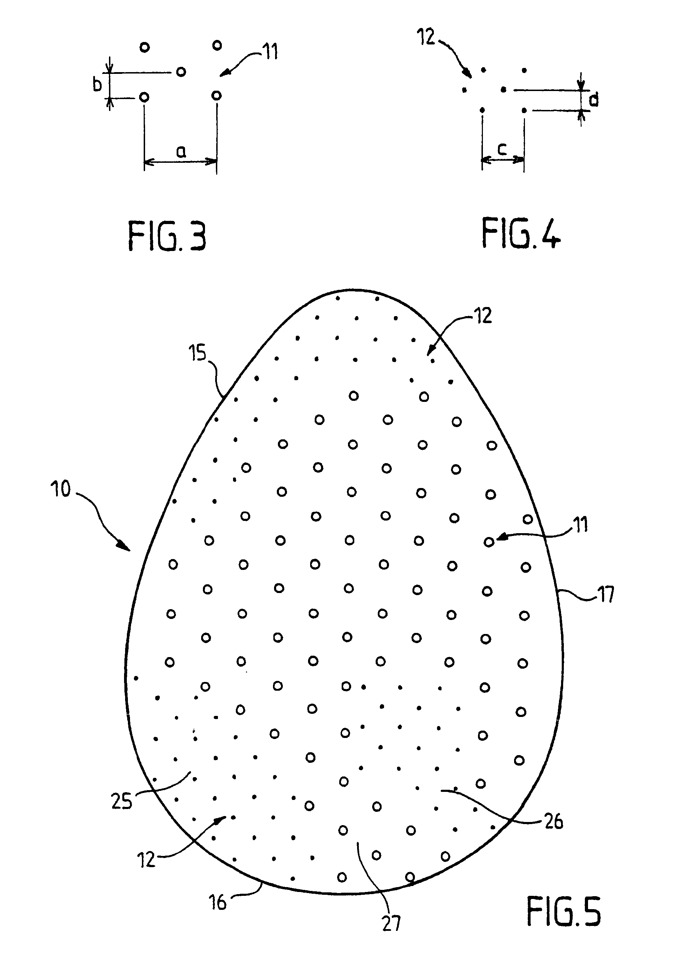

Par ailleurs, les perforations de colonisation 11 et les perforations de mise en place 12 sont réparties selon des rangées s'étendant sensiblement perpendiculairement à l'axe de symétrie longitudinal de la plaque, les perforations d'une rangée étant décalées par rapport aux perforations de la rangée suivante. Ainsi, les perforations de colonisation 11 sont écartées les unes des autres dans une même rangée d'un écart a voisin de 18 mm (figure 3) tandis que deux rangées successives sont espacées d'un écart b voisin de 6 mm. De même, les perforations de mise en place 12 sont espacées dans une même rangée (figure 4) d'un écart c voisin de 10 mm tandis que deux rangées successives sont écartées d'un écart b voisin de 5 mm.

La plaque de renfort 10 est réalisée par découpage laser d'une laize non tissée réalisée en polypropylène. Les perforations de colonisation 11 et les perforations de mise en place 12 étant ensuite réalisées par découpage aux rayons laser ou par découpage aux ultra sons.

La plaque de renfort 10 est d'épaisseur sensiblement constante et est souple de manière à pouvoir être enroulée sur elle-même et être introduite dans le corps du patient lorsque la technique par laparoscopie est utilisée. De plus, cette plaque possède une certaine mémoire de forme, de sorte que la plaque de renfort 10 retrouve une forme sensiblement plane lorsqu'elle est mise en place dans l'abdomen du patient.

Dans le deuxième mode de réalisation de l'invention représenté à la figure 5, seule la répartition des perforations de mise en place 12 situées dans la partie inférieure 16 de la plaque de renfort 10 est différente de celle du premier mode de réalisation.

Dans la partie inférieure 16, les perforations de mise en place 12 sont réparties en deux zones 25 et 26 entre lesquelles s'étendent des perforations de colonisation 11 dans une bande 27.

Dans ce mode de réalisation, la répartition des perforations de mise en place 12 permet encore mieux de mettre en évidence les zones dans lesquelles le chirurgien peut pratiquer les points de suture ou l'agrafage.

Bien entendu, la plaque de renfort 10 peut être de forme géométrique différente, par exemple rectangulaire.

De plus, la plaque de renfort selon la présente invention peut être utilisée pour résorber d'autres types de hernies tels que par exemple, les hernies inguinales-crurales, les hernies ombilicales, les hernies incisionnelles ou bien encore les éventrations.

### POSSIBILITES D'APPLICATION INDUSTRIELLE

L'invention trouve son application industrielle dans la fabrication d'implants herniaires.

## Revendications

1. Plaque de renfort (10) pour le traitement de tissus altérés (5), ladite plaque (10) comportant des perforations (11) pour favoriser la colonisation cellulaire et la reconstitution des tissus altérés (5) et étant **caractérisée en ce qu'**elle est formée d'un matériau non tissé à base de polypropylène et **en ce qu'**elle comprend en outre des perforations (12) favorisant la mise en place de la plaque (10) en regard des tissus altérés (5), les perforations de colonisation (11) étant de dimensions différentes des perforations de mise en place (12).

2. Plaque de renfort selon la revendication 1, **caractérisée en ce que** les perforations de colonisation (11) sont de dimensions supérieures à celles des perforations de mise en place (12).

3. Plaque de renfort selon l'une des revendications 1 ou 2, **caractérisée en ce que** les perforations de colonisation (11) sont de forme sensiblement ovale.

4. Plaque de renfort selon l'une quelconque des revendications 1 à 3, **caractérisée en ce que** les perforations de mise en place (12) sont de forme sensiblement circulaire.

5. Plaque de renfort selon l'une quelconque des revendications 1 à 4, **caractérisée en ce que** la plaque (10) est de forme sensiblement ovale et comporte une portion supérieure rétrécie (15), une portion inférieure élargie (16) et une portion centrale intermédiaire (17), les perforations de mise en place (12) étant uniformément réparties dans les portions élargie (16) et rétrécie (15), et les perforations de colonisation (11) étant uniformément réparties dans la portion intermédiaire (17).

6. Plaque de renfort selon la revendication 5, **caractérisée en ce que** la densité des perforations de mise en place (12) des portions élargie (16) et rétrécie (15) est supérieure à la densité des perforations de colonisation (11) de la portion intermédiaire (17).

7. Plaque de renfort selon la revendication 5 ou 6, **caractérisée en ce que** la portion élargie (16) comprend en outre des perforations de colonisation (11) qui s'étendent entre deux zones (25,26) de perforations de mise en place (12).

8. Plaque de renfort selon l'une quelconque des revendications 1 à 7, **caractérisée en ce que** les perforations de colonisation (11) et les perforations de mise en place (12) sont réalisées par découpage laser.

9. Plaque de renfort selon l'une quelconque des revendications 1 à 8, **caractérisée en ce que** la plaque (10) est souple et d'épaisseur sensiblement constante.

10. Plaque de renfort selon la revendication 9, **caractérisée en ce que** la plaque (10) est réalisée en matériau polypropylène possédant une certaine mémoire de forme pour s'étendre sensiblement dans un plan après avoir été enroulée sur elle-même.

11. Plaque de renfort selon l'une quelconque des revendications 1 à 10, **caractérisée en ce qu'**elle constitue une plaque de renfort pour le traitement de tissus altérés (5) de la paroi abdominale (2).

12. Plaque de renfort selon l'une quelconque des revendications 1 à 11, **caractérisée en ce qu'**elle constitue une plaque de renfort pour le traitement des hernies, des prolapsus ou des éventrations.

## Claims

1. Reinforcement plate (10) for treating impaired tissues (5), the said plate (10) comprising perforations (11) for promoting cell colonisation and the reconstitution of the impaired tissues (5) and being **characterised in that** it is formed from a non-woven material based on polypropylene and **in that** it also comprises perforations (12) assisting the positioning of the plate (10) opposite the impaired tissues (5), the colonisation perforations (11) being of different dimensions from the positioning perforations (12),

2. Reinforcement plate according to claim 1, **characterised in that** the colonisation perforations (11) have dimensions greater than those of the positioning perforations (12).

3. Reinforcement plate according to one of claim 1 or 2, **characterised in that** the colonisation perforations (11) have a substantially oval shape.

4. Reinforcement plate according to any one of claims 1 to 3, **characterised in that** the positioning perforations (12) have a substantially circular shape.

5. Reinforcement plate according to any one of claims 1 to 4, **characterised in that** the plate (10) has a substantially oval shape and comprises a narrowed top portion (15), a broadened bottom portion (16) and an intermediate central portion (17), the positioning perforations (12) being uniformly distributed in the broadened (16) and narrowed (15) portions, and the colonisation perforations (11) being uniformly distributed in the intermediate portion (17).

6. Reinforcement plate according to claim 5, **characterised in that** the density of the positioning perforations (12) of the broadened (16) and narrowed (15) portions is greater than the density of the colonisation perforations (11) of the intermediate portion (17).

7. Reinforcement plate according to claim 5 or 6, **characterised in that** the broadened portion (16) also comprises colonisation perforations (11) that extend between two areas (25, 26) of positioning perforations (12).

8. Reinforcement plate according to any one of claims 1 to 7, **characterised in that** the colonisation perforations (11) and the positioning perforations (12) are produced by laser cutting.

9. Reinforcement plate according to any one of claims 1 to 8, **characterised in that** the plate (10) is flexible and of substantially constant thickness.

10. Reinforcement plate according to claim 9, **characterised in that** the plate (10) is produced from polypropylene material having a certain shape memory so as to extend substantially in one plane after having been wound on itself.

11. Reinforcement plate according to any one of claims 1 to 10, **characterised in that** it constitutes a reinforcement plate for the treatment of impaired tissues (5) of the abdominal wall (2).

12. Reinforcement plate according to any one of claims 1 to 11, **characterised in that** it constitutes a reinforcement plate for the treatment of hernias, prolapses or ventral ruptures.

## Patentansprüche

1. Versteifungsplatte (10) zur Behandlung von geschädigtem Gewebe (5), wobei die Platte (10) Löcher (11) umfasst, um die Zellbesiedelung und die Wiederherstellung des geschädigten Gewebes (5) zu begünstigen und **dadurch gekennzeichnet, dass** sie aus einem Vliesstoff auf der Basis von Polypropylen besteht und dadurch, dass sie darüber hinaus Löcher (12) umfasst, die die Anordnung der Platte (10) in Bezug auf das geschädigte Gewebe (5) begünstigen, wobei sich die Abmessungen der Besiedlungslöcher (11) von denen der Anordnungslöcher (12) unterscheiden.

2. Versteifungsplatte nach Anspruch 1, **dadurch gekennzeichnet, dass** die Abmessungen der Besiedlungslöcher (11) größer als die der Anordnungslöcher (12) sind.

3. Versteifungsplatte nach einem der Ansprüche 1 oder 2, **dadurch gekennzeichnet, dass** die Besiedlungslöcher (11) im Wesentlichen oval geformt sind.

4. Versteifungsplatte nach einem der Ansprüche 1 bis 3, **dadurch gekennzeichnet, dass** die Anordnungslöcher (12) im Wesentlichen kreisförmig sind.

5. Versteifungsplatte nach einem der Ansprüche 1 bis 4, **dadurch gekennzeichnet, dass** die Platte (10) im Wesentlichen oval geformt ist und einen oberen verengten Abschnitt (15), einen unteren verbreiterten Abschnitt (16) und einen dazwischen liegenden mittleren Abschnitt (17) umfasst, wobei die Anordnungslöcher (12) gleichmäßig im verbreiterten Abschnitt (16) und im verengten Abschnitt (15) verteilt sind und die Besiedlungslöcher (11) gleichmäßig im dazwischen liegenden Abschnitt (17) verteilt sind.

6. Versteifungsplatte nach Anspruch 5, **dadurch gekennzeichnet, dass** die Dichte der Anordnungslöcher (12) im verbreiterten (16) und verengten Abschnitt (15) höher ist als die Dichte der Besiedlungslöcher (11) im dazwischen liegenden Abschnitt (17).

7. Versteifungsplatte nach Anspruch 5 oder 6, **dadurch gekennzeichnet, dass** der verbreiterte Abschnitt (16) darüber hinaus Besiedlungslöcher (11) umfasst, die sich zwischen zwei Bereichen (25, 26) von Anordnungslöchern (12) erstrecken.

8. Versteifungsplatte nach einem der Ansprüche 1 bis 7, **dadurch gekennzeichnet, dass** die Besiedlungslöcher (11) und die Anordnungslöcher (12) durch Laserschneiden hergestellt werden.

9. Versteifungsplatte nach einem der Ansprüche 1 bis 8, **dadurch gekennzeichnet, dass** die Platte (10) flexibel ist und eine im Wesentlichen konstante Dicke aufweist.

10. Versteifungsplatte nach Anspruch 9, **dadurch gekennzeichnet, dass** die Platte (10) aus einem Polypropylenmaterial mit einem gewissen Formgedächtnis besteht, damit sie sich im Wesentlichen in einer Ebene ausstrecken kann, nachdem sie zusammengerollt wurde.

11. Versteifungsplatte nach einem der Ansprüche 1 bis 10, **dadurch gekennzeichnet, dass** sie eine Versteifungsplatte zur Behandlung von geschädigtem Gewebe (5) der Bauchwand (2) bildet.

12. Versteifungsplatte nach einem der Ansprüche 1 bis 11, **dadurch gekennzeichnet, dass** sie eine Versteifungsplatte zur Behandlung von Hernien, Vorfällen oder Eventrationen bildet.
